# EUROPEAN PATENT APPLICATION

(11) **EP 3 889 256 A1**
(43) Date of publication of application: **06.10.2021**
(21) Application number: 18941327.1
(22) Date of filing: 30.11.2018
(51) Int. Cl.: C12N 15/09, C12N 1/21, C12N 15/53, C12P 7/56

(54) **LACTIC ACID-PRODUCING TRANSFORMANT OF BACTERIUM OF GENUS HYDROGENOPHILUS**

(71) Applicant: Utilization of Carbon Dioxide Institute Co.,Ltd., Tokyo, 108-0014 (JP)
(72) Inventor: YUKAWA Hideaki, Tokyo 108-0014 (JP); OHTANI Naoto, Tokyo 108-0014 (JP)
(74) Representative: Lederer & Keller Patentanwälte Partnerschaft mbB
(86) International application number: PCT/JP2018/044226
(87) International publication number: WO 2020/110300

(57) **Abstract**

A transformant obtained by introducing (a) a lactate dehydrogenase gene and/or (b) a malate/lactate dehydrogenase gene into a *Hydrogenophilus* bacterium efficiently produces lactic acid through use of carbon dioxide as a sole carbon source. *Parageobacillus thermoglucosidasius* ldh gene, *Geobacillus kaustophilus* ldh gene and *Thermus thermophilus* ldh gene of lactate dehydrogenases, and *Thermus thermophilus* mldh gene and *Meiothermus ruber* mldh-1 and mldh-2 genes of malate/lactate dehydrogenases are preferable in that they have good lactic acid production efficiency.

SEQUENCE LISTING

<110>  Utilization of Carbon Dioxide Institute Co.,Ltd.

<120>  Hydrogenophilus bacterium transformant producing lactic acid

<130>  FPR0013WO

<160>  34

<170>  PatentIn version 3.5

<210>  1
<211>  960
<212>  DNA
<213>  Parageobacillus thermoglucosidasius

<400>  1
atgaaacaac aaggcatgaa tcgagtagca cttataggaa cggggttcgt tggggccagc      60

tatgcatttg cccttatgaa ccaaggaata gcagatgagt tagtattgat tgatgtaaat     120

aagaataagg cagagggcga tgtgatggat ttaaatcacg gaaaagtatt cgcgccgaag     180

ccgatgaata tttggtttgg agattatcaa gattgccaag acgccgattt ggtggtgatt     240

tgtgcagggg ctaaccaaaa gccgggagaa acaagactgg atcttgttga caaaaatatt     300

aatatcttca aaacgattgt cgattctgtg atgaaatccg gatttgatgg cgtttttctt     360

gtggcaacga acccagtgga tattttaacg tatgctactt ggaaatttag cgggttaccg     420

aaagagcggg taatcggctc aggaacgatt cttgatacag caagattccg cttcttgcta     480

agtgaatatt ttcaagtggc tccgaccaat gtacatgcgt atattattgg cgagcatggg     540

gatacagagc tgcctgtttg gagccatgcg gaaattggaa gcattccagt tgagcaaata     600

ttgatgcaaa acgataacta tagaaaagag gatttagaca atatctttgt taatgttcgt     660

gatgcggcat atcaaatcat tgagaaaaaa ggggcaacgt attacggcat tgcaatggga     720

ttagtccgta tcactcgtgc tattttgcac aatgaaaatg ccatcttaac cgtttctgct     780

catttggacg gccaatatgg cgaacgaaat gtttatattg gcgtgcctgc cattatcaac     840

cgaaacggta ttcgtgaagt gatggaattg acgctaaatg aaacagaaca acaacaattc     900

catcatagtg taactgtatt aaaagacatt ctttcccgtt attttgatga tgtaaaataa     960


<210>  2
<211>  954
<212>  DNA
<213>  Geobacillus kaustophilus

<400>  2
atgaaaaacg ggagaggaaa tcgggtagcg gtcgtcggca ccgggtttgt cggcgccagt      60

tatgcgtttg ccttaatgaa tcaagggatt gccgatgaga tcgtgctcat cgatgcaaat     120

gaaaacaagg ctgagggcga tgcgatggac ttcaaccatg ggaaagtatt tgcgccgaag     180

ccggctgaca tttggcacgg cgattacgat gattgccgcg atgccgattt ggttgtcatt 240

tgcgccggcg ccaaccaaaa accgggcgag acgcggcttg atcttgtgga caaaaacatt 300

gccattttcc gctcgatcgt tgagtcggtc atggcatccg gatttcaagg actgtttctc 360

gtcgccacca atccggtcga cattttaacg tacgcgacgt ggaaattcag cggcctgccg 420

caagagcgag taatcggatc gggcacgatt ttggacacgg cgcggttccg cttcttgttg 480

ggcgactatt tcgccgtcgc cccgacgaac gtgcacgcct atattatcgg cgaacatggc 540

gacactgaac tcccggtctg gagccaggct gatatcggcg gcgtgccgat ccgcaagctg 600

gtcgagtcta aaggggaaga agcgcaaaaa gagctcgagc gcatttttgt caatgtgcgc 660

gatgccgcct accaaattat tgagaaaaaa ggagcgacgt actacgggat tgctatgggg 720

cttgcccgcg tgacgcgcgc cattttgcat catgaaaatg ccattttgac cgtttccgct 780

tacttggacg gcccatacgg cgaacgcgat gtctacatcg gtgtgcctgc tgtgatcaac 840

cgaaatggca tccgcgaagt gattgaaatt gaacttgacg aggaggagaa aaaatggttc 900

caccgtagtg ctgcgacgtt aaaaggtgta ttggcgcgct attttgctca gtaa 954

<210> 3
<211> 933
<212> DNA
<213> Thermus thermophilus

<400> 3
atgaaggtcg gcatcgtggg aagcggcatg gtggggagcg ccaccgccta cgccctggcc 60

ctcctcggcg tggcgcggga ggtggtcctc gtggacctgg accggaagct ggcccaggcc 120

cacgccgagg acatcctcca cgccacgccc ttcgcccacc cggtctgggt gcgggcgggg 180

tcgtacgggg acctcgaggg ggcccgggcg gtggtgctcg ccgccggggt ggcccagcgc 240

cccggggaga cccgcctgca gcttctggac cgcaacgccc aggtcttcgc ccaggtggtg 300

ccccgggttt tagaggcggc cccggaggcg gtgctcctcg tggccacgaa cccggtggac 360

gtgatgaccc aggtggccta ccgcctctcc ggcctgcccc cggggcgggt ggtgggctcg 420

gggacgatcc tggacacggc ccgcttccgg gcccttctgg cggagtacct ccgggtggcc 480

ccccagtcgg tccacgccta cgtgctgggg gagcacgggg actcggaggt gctggtctgg 540

tccagcgccc aggtgggcgg ggtgcccctc ctggagttcg ccgaggcccg ggggcggccc 600

ctttccccgg aggaccgggc ccgcattgac gaaggggtcc gccgggccgc ctaccggatc 660

attgagggga agggggccac ctactacggc atcggggcgg gcctcgcccg gcttgtgcgg 720

gccatcctca ccgacgaaaa gggggtgtac accgtgagcg ccttcacccc cgaggtggag 780

ggggtcttgg aggtgagcct ctccctgccc cgcatcctgg gcgcgggggg cgtggagggg 840

accgtctacc cgagcctgag cccggaggag cgggaggcct tgcggcggag cgccgagatc 900

ctcaaggagg cggccttcgc cctggggttt tag                                    933

<210>  4
<211>  319
<212>  PRT
<213>  Parageobacillus thermoglucosidasius

<400>  4

Met Lys Gln Gln Gly Met Asn Arg Val Ala Leu Ile Gly Thr Gly Phe
1               5                   10                  15

Val Gly Ala Ser Tyr Ala Phe Ala Leu Met Asn Gln Gly Ile Ala Asp
            20                  25                  30

Glu Leu Val Leu Ile Asp Val Asn Lys Asn Lys Ala Glu Gly Asp Val
        35                  40                  45

Met Asp Leu Asn His Gly Lys Val Phe Ala Pro Lys Pro Met Asn Ile
    50                  55                  60

Trp Phe Gly Asp Tyr Gln Asp Cys Gln Asp Ala Asp Leu Val Val Ile
65                  70                  75                  80

Cys Ala Gly Ala Asn Gln Lys Pro Gly Glu Thr Arg Leu Asp Leu Val
                85                  90                  95

Asp Lys Asn Ile Asn Ile Phe Lys Thr Ile Val Asp Ser Val Met Lys
            100                 105                 110

Ser Gly Phe Asp Gly Val Phe Leu Val Ala Thr Asn Pro Val Asp Ile
        115                 120                 125

Leu Thr Tyr Ala Thr Trp Lys Phe Ser Gly Leu Pro Lys Glu Arg Val
    130                 135                 140

Ile Gly Ser Gly Thr Ile Leu Asp Thr Ala Arg Phe Arg Phe Leu Leu
145                 150                 155                 160

Ser Glu Tyr Phe Gln Val Ala Pro Thr Asn Val His Ala Tyr Ile Ile
                165                 170                 175

Gly Glu His Gly Asp Thr Glu Leu Pro Val Trp Ser His Ala Glu Ile
            180                 185                 190

Gly Ser Ile Pro Val Glu Gln Ile Leu Met Gln Asn Asp Asn Tyr Arg
        195                 200                 205

Lys Glu Asp Leu Asp Asn Ile Phe Val Asn Val Arg Asp Ala Ala Tyr

210                 215                 220

Gln Ile Ile Glu Lys Lys Gly Ala Thr Tyr Tyr Gly Ile Ala Met Gly
225                 230                 235                 240

Leu Val Arg Ile Thr Arg Ala Ile Leu His Asn Glu Asn Ala Ile Leu
                245                 250                 255

Thr Val Ser Ala His Leu Asp Gly Gln Tyr Gly Glu Arg Asn Val Tyr
            260                 265                 270

Ile Gly Val Pro Ala Ile Ile Asn Arg Asn Gly Ile Arg Glu Val Met
        275                 280                 285

Glu Leu Thr Leu Asn Glu Thr Glu Gln Gln Gln Phe His His Ser Val
    290                 295                 300

Thr Val Leu Lys Asp Ile Leu Ser Arg Tyr Phe Asp Asp Val Lys
305                 310                 315

<210>  5
<211>  317
<212>  PRT
<213>  Geobacillus kaustophilus

<400>  5

Met Lys Asn Gly Arg Gly Asn Arg Val Ala Val Val Gly Thr Gly Phe
1               5                   10                  15

Val Gly Ala Ser Tyr Ala Phe Ala Leu Met Asn Gln Gly Ile Ala Asp
            20                  25                  30

Glu Ile Val Leu Ile Asp Ala Asn Glu Asn Lys Ala Glu Gly Asp Ala
        35                  40                  45

Met Asp Phe Asn His Gly Lys Val Phe Ala Pro Lys Pro Ala Asp Ile
    50                  55                  60

Trp His Gly Asp Tyr Asp Asp Cys Arg Asp Ala Asp Leu Val Val Ile
65                  70                  75                  80

Cys Ala Gly Ala Asn Gln Lys Pro Gly Glu Thr Arg Leu Asp Leu Val
                85                  90                  95

Asp Lys Asn Ile Ala Ile Phe Arg Ser Ile Val Glu Ser Val Met Ala
            100                 105                 110

Ser Gly Phe Gln Gly Leu Phe Leu Val Ala Thr Asn Pro Val Asp Ile

115                 120                 125

Leu Thr Tyr Ala Thr Trp Lys Phe Ser Gly Leu Pro Gln Glu Arg Val
    130                 135                 140

Ile Gly Ser Gly Thr Ile Leu Asp Thr Ala Arg Phe Arg Phe Leu Leu
145                 150                 155                 160

Gly Asp Tyr Phe Ala Val Ala Pro Thr Asn Val His Ala Tyr Ile Ile
                165                 170                 175

Gly Glu His Gly Asp Thr Glu Leu Pro Val Trp Ser Gln Ala Asp Ile
            180                 185                 190

Gly Gly Val Pro Ile Arg Lys Leu Val Glu Ser Lys Gly Glu Glu Ala
        195                 200                 205

Gln Lys Glu Leu Glu Arg Ile Phe Val Asn Val Arg Asp Ala Ala Tyr
    210                 215                 220

Gln Ile Ile Glu Lys Lys Gly Ala Thr Tyr Tyr Gly Ile Ala Met Gly
225                 230                 235                 240

Leu Ala Arg Val Thr Arg Ala Ile Leu His His Glu Asn Ala Ile Leu
                245                 250                 255

Thr Val Ser Ala Tyr Leu Asp Gly Pro Tyr Gly Glu Arg Asp Val Tyr
            260                 265                 270

Ile Gly Val Pro Ala Val Ile Asn Arg Asn Gly Ile Arg Glu Val Ile
        275                 280                 285

Glu Ile Glu Leu Asp Glu Glu Glu Lys Lys Trp Phe His Arg Ser Ala
    290                 295                 300

Ala Thr Leu Lys Gly Val Leu Ala Arg Tyr Phe Ala Gln
305                 310                 315

<210>  6
<211>  310
<212>  PRT
<213>  Thermus thermophilus

<400>  6

Met Lys Val Gly Ile Val Gly Ser Gly Met Val Gly Ser Ala Thr Ala
1               5                   10                  15

Tyr Ala Leu Ala Leu Leu Gly Val Ala Arg Glu Val Val Leu Val Asp

20                  25                  30

Leu Asp Arg Lys Leu Ala Gln Ala His Ala Glu Asp Ile Leu His Ala
        35                  40                  45

Thr Pro Phe Ala His Pro Val Trp Val Arg Ala Gly Ser Tyr Gly Asp
    50                  55                  60

Leu Glu Gly Ala Arg Ala Val Val Leu Ala Ala Gly Val Ala Gln Arg
65                  70                  75                  80

Pro Gly Glu Thr Arg Leu Gln Leu Leu Asp Arg Asn Ala Gln Val Phe
                85                  90                  95

Ala Gln Val Val Pro Arg Val Leu Glu Ala Ala Pro Glu Ala Val Leu
            100                 105                 110

Leu Val Ala Thr Asn Pro Val Asp Val Met Thr Gln Val Ala Tyr Arg
        115                 120                 125

Leu Ser Gly Leu Pro Pro Gly Arg Val Val Gly Ser Gly Thr Ile Leu
    130                 135                 140

Asp Thr Ala Arg Phe Arg Ala Leu Leu Ala Glu Tyr Leu Arg Val Ala
145                 150                 155                 160

Pro Gln Ser Val His Ala Tyr Val Leu Gly Glu His Gly Asp Ser Glu
                165                 170                 175

Val Leu Val Trp Ser Ser Ala Gln Val Gly Gly Val Pro Leu Leu Glu
            180                 185                 190

Phe Ala Glu Ala Arg Gly Arg Ala Leu Ser Pro Glu Asp Arg Ala Arg
        195                 200                 205

Ile Asp Glu Gly Val Arg Arg Ala Ala Tyr Arg Ile Ile Glu Gly Lys
    210                 215                 220

Gly Ala Thr Tyr Tyr Gly Ile Gly Ala Gly Leu Ala Arg Leu Val Arg
225                 230                 235                 240

Ala Ile Leu Thr Asp Glu Lys Gly Val Tyr Thr Val Ser Ala Phe Thr
                245                 250                 255

Pro Glu Val Glu Gly Val Leu Glu Val Ser Leu Ser Leu Pro Arg Ile
            260                 265                 270

Leu Gly Ala Gly Gly Val Glu Gly Thr Val Tyr Pro Ser Leu Ser Pro
        275                 280                 285

Glu Glu Arg Glu Ala Leu Arg Arg Ser Ala Glu Ile Leu Lys Glu Ala
    290                 295                 300

Ala Phe Ala Leu Gly Phe
305                 310

<210>  7
<211>  1035
<212>  DNA
<213>  Thermus thermophilus

<400>  7
atgaggtggc gggcggactt cctctcggcc tgggcggagg ccctcttgcg aaaggcggga      60

gcggacgaac cctccgccaa ggcggtggcc tgggccctgg tggaggcgga cctcaggggg     120

gtgggaagcc acgggctttt gcgccttccc gtttacgtgc gccgcctcga ggcgggcctg     180

gtgaacccca gccccaccct gcccctggag gaacggggcc ccgtggccct cctggacggg     240

gagcacggct tcggaccccg cgtggcccta aaggccgtgg aggcggccca aagcctcgca     300

aggaggcacg gcctcggggc cgtgggggtg cggcggagca cccacttcgg catggcgggc     360

ctctacgcgg agaagctcgc ccgggagggc ttcgtggcct gggtcaccac caacgccgag     420

cccgacgtgg tgcccttcgg ggggcgggag aaggccttgg gcaccaaccc tctggccttc     480

gccgccccgg cccctcaggg gatcctcgtg gccgacctgg ccacctcgga aagcgccatg     540

ggcaaggtct tcctagcccg ggagaagggg gagcggatcc ccccaagctg gggggtggac     600

cgggagggga gccccacgga cgacccccac cgggtctacg ccctgaggcc cctcgggggg     660

cccaaggggt acgccctggc ccttttggtg gaggtgctct cgggggtgct cacgggggcg     720

ggggtggccc acggcatcgg ccgcatgtac gacgagtggg accgccccca ggacgtgggc     780

cacttcctcc tggccctgga cccggggcgc ttcgtgggca aagaggcctt cctggagcgg     840

atgggggccc tttggcaagc cctaaaggcc actcccccgg cgccggggca cgaggaggtc     900

ttcctccccg gggagttgga ggccaggagg cgggagcggg ccctggcgga ggggatggcc     960

cttccggagc gggtggtggc ggagcttaag gccttggggg agcgctacgg cgtgccttgg    1020

agggacgatg cttga                                                     1035

<210>  8
<211>  1011
<212>  DNA
<213>  Meiothermus ruber

<400>  8
atgcaaggca ttcctgtgca acaactgcgc gagcgggtgg agcagattct aataaaccgg      60

ggctttacgc tggagaatgc tctacccatc gcagaatccc tggtgctggc cgagatgcgg 120

ggggttgcct cgcacggcct gatccgactg cccatctacc tcgagcgcgc ccgactgggt 180

tcggtaaaac cccaggcccg gcccgtgctg ctggcggatt atccagccct ggccctgctg 240

gatgcccagg atggtcacgg catcccctcc ggcttgaaag cgatggagct ggccattgaa 300

aaagcccaga aggtgggcct ggccgctgtg ggggtgcggc gctcgagcca ctttggcctg 360

gcctggtact tcgtgcgcag cgcagtggaa aaggggctgg tcggcgtggc actctccaac 420

gccgatgcgc tggtggcccc ctggggcgcc cgcagccgct ttctgggcac caaccccctg 480

gctgtgggca tcccggccat ggaggaaccc cccatcgccc tggacatggc caccagcgag 540

gccgcccacg gcaaaatttt gctggccaag tccagcggga aaaccatccc cctcaactgg 600

gccctcgatg cggagggcgg gcccaccgac gaccccgacc gggccctggc cggcgccctg 660

ctgccttttg gggggcccaa gggatcggcc atcagcctgc tcattgatgt gctgtgcggc 720

ccactcgtgg gcgctctgat tggccccgag atcgccccgc tctacaccga gcccgaacgg 780

ccccagggcc tgggccattt ttttatggcc ctgaacccgg gtgtttttgg cgacgccgaa 840

cagtttagaa agcaggtcga cgcgtacatt cgcagggttc gcgcgctgcc tcccgccgaa 900

aacgtcgatc gggttctact gccaggcgaa cgcgagtggc gcctcgagca aaaagcgcta 960

caggaggggg tgtctctaag cccagaggcc gctaaagcgg tgggccttta a 1011

<210> 9
<211> 999
<212> DNA
<213> Meiothermus ruber

<400> 9
atgagggttc cttatcccgt actcaagcag gcggtctcga gccacttcca gggcctgggg 60

ctggccccgg atcatgccga ggccttcacc gaggtgatcc tcgaggccga gctcgagggc 120

aacctggggc acggcctgac ccggattgcc cagtacaccg cccagctaca ggccggtggg 180

ctcaaccccc ggccgcagat gcgtttggaa cgaaccaaac ccggggttgc agttctgcat 240

gccgacggcg cacccgggcc ggtggccggg ctttttgcag tgcaggcgct ggccccgatg 300

gccagggagc agggaagcgc cgccctggcc gtgcgcggcg cggggcattc cggggtgctc 360

tcggcgtacg tgggccggct ggcccaagag ggcctggtag ccctggcctt tgccaacacc 420

cccccggcca tcgccccggg gccggtgctg ggcaccaacc ccatcgccct gggcgcgccg 480

gccgagcccc agccggtcat cattgatacc tccatctcgg tggtggcgcg cggcaagatc 540

atcgccgcgg ctaaaaaggg cgagcccatc ccgccgggct gggcgctcga caaggagggt 600

cgcccaacca ccgatgccaa ggctgcgctg gaaggctcac tgctgcccat tggcgagggc 660

aaggggtttg cgctggcagt gctggtggaa attctggccg gggccctggc gggcgacgtg 720

ctctcgcccg agctgcccct gccctggatg cccccagcgc aggccgccaa gccggggctg 780

ctgctgctgg cctttgaccc cgccgccttt ggcccgggct acaggggccg ggtggcccag 840

ctcatcgagg ctcttaaagc ggccggaggc cggattcccg gtgcgcgccg ggccgcttta 900

cgagagaaag ccttggcgga aggtctggag gtcaaccaga cgcttcaggc cgaactcggt 960

acactaggcg tgcatctaca aggaggaggg acaagatga 999

<210> 10
<211> 344
<212> PRT
<213> Thermus thermophilus

<400> 10

Met Arg Trp Arg Ala Asp Phe Leu Ser Ala Trp Ala Glu Ala Leu Leu
1               5                   10                  15

Arg Lys Ala Gly Ala Asp Glu Pro Ser Ala Lys Ala Val Ala Trp Ala
            20                  25                  30

Leu Val Glu Ala Asp Leu Arg Gly Val Gly Ser His Gly Leu Leu Arg
        35                  40                  45

Leu Pro Val Tyr Val Arg Arg Leu Glu Ala Gly Leu Val Asn Pro Ser
    50                  55                  60

Pro Thr Leu Pro Leu Glu Glu Arg Gly Pro Val Ala Leu Leu Asp Gly
65                  70                  75                  80

Glu His Gly Phe Gly Pro Arg Val Ala Leu Lys Ala Val Glu Ala Ala
                85                  90                  95

Gln Ser Leu Ala Arg Arg His Gly Leu Gly Ala Val Gly Val Arg Arg
            100                 105                 110

Ser Thr His Phe Gly Met Ala Gly Leu Tyr Ala Glu Lys Leu Ala Arg
        115                 120                 125

Glu Gly Phe Val Ala Trp Val Thr Thr Asn Ala Glu Pro Asp Val Val
    130                 135                 140

Pro Phe Gly Gly Arg Glu Lys Ala Leu Gly Thr Asn Pro Leu Ala Phe
145                 150                 155                 160

Ala Ala Pro Ala Pro Gln Gly Ile Leu Val Ala Asp Leu Ala Thr Ser
                165                 170                 175

Glu Ser Ala Met Gly Lys Val Phe Leu Ala Arg Glu Lys Gly Glu Arg

180                 185                 190

Ile Pro Pro Ser Trp Gly Val Asp Arg Glu Gly Ser Pro Thr Asp Asp
        195                 200                 205

Pro His Arg Val Tyr Ala Leu Arg Pro Leu Gly Gly Pro Lys Gly Tyr
    210                 215                 220

Ala Leu Ala Leu Leu Val Glu Val Leu Ser Gly Val Leu Thr Gly Ala
225                 230                 235                 240

Gly Val Ala His Gly Ile Gly Arg Met Tyr Asp Glu Trp Asp Arg Pro
                245                 250                 255

Gln Asp Val Gly His Phe Leu Leu Ala Leu Asp Pro Gly Arg Phe Val
            260                 265                 270

Gly Lys Glu Ala Phe Leu Glu Arg Met Gly Ala Leu Trp Gln Ala Leu
        275                 280                 285

Lys Ala Thr Pro Pro Ala Pro Gly His Glu Glu Val Phe Leu Pro Gly
    290                 295                 300

Glu Leu Glu Ala Arg Arg Arg Glu Arg Ala Leu Ala Glu Gly Met Ala
305                 310                 315                 320

Leu Pro Glu Arg Val Val Ala Glu Leu Lys Ala Leu Gly Glu Arg Tyr
                325                 330                 335

Gly Val Pro Trp Arg Asp Asp Ala
            340

<210>  11
<211>  336
<212>  PRT
<213>  Meiothermus ruber

<400>  11

Met Gln Gly Ile Pro Val Gln Gln Leu Arg Glu Arg Val Glu Gln Ile
1               5                   10                  15

Leu Ile Asn Arg Gly Phe Thr Leu Glu Asn Ala Leu Pro Ile Ala Glu
            20                  25                  30

Ser Leu Val Leu Ala Glu Met Arg Gly Val Ala Ser His Gly Leu Ile
        35                  40                  45

Arg Leu Pro Ile Tyr Leu Glu Arg Ala Arg Leu Gly Ser Val Lys Pro

50                  55                  60

Gln Ala Arg Pro Val Leu Leu Ala Asp Tyr Pro Ala Leu Ala Leu Leu
65                  70                  75                  80

Asp Ala Gln Asp Gly His Gly Ile Pro Ser Gly Leu Lys Ala Met Glu
                85                  90                  95

Leu Ala Ile Glu Lys Ala Gln Lys Val Gly Leu Ala Ala Val Gly Val
            100                 105                 110

Arg Arg Ser Ser His Phe Gly Leu Ala Trp Tyr Phe Val Arg Ser Ala
        115                 120                 125

Val Glu Lys Gly Leu Val Gly Val Ala Leu Ser Asn Ala Asp Ala Leu
    130                 135                 140

Val Ala Pro Trp Gly Ala Arg Ser Arg Phe Leu Gly Thr Asn Pro Leu
145                 150                 155                 160

Ala Val Gly Ile Pro Ala Met Glu Glu Pro Pro Ile Ala Leu Asp Met
                165                 170                 175

Ala Thr Ser Glu Ala Ala His Gly Lys Ile Leu Leu Ala Lys Ser Ser
            180                 185                 190

Gly Lys Thr Ile Pro Leu Asn Trp Ala Leu Asp Ala Glu Gly Arg Pro
        195                 200                 205

Thr Asp Asp Pro Asp Arg Ala Leu Ala Gly Ala Leu Leu Pro Phe Gly
    210                 215                 220

Gly Pro Lys Gly Ser Ala Ile Ser Leu Leu Ile Asp Val Leu Cys Gly
225                 230                 235                 240

Pro Leu Val Gly Ala Leu Ile Gly Pro Glu Ile Ala Pro Leu Tyr Thr
                245                 250                 255

Glu Pro Glu Arg Pro Gln Gly Leu Gly His Phe Phe Met Ala Leu Asn
            260                 265                 270

Pro Gly Val Phe Gly Asp Ala Glu Gln Phe Arg Lys Gln Val Asp Ala
        275                 280                 285

Tyr Ile Arg Arg Val Arg Ala Leu Pro Pro Ala Glu Asn Val Asp Arg
    290                 295                 300

Val Leu Leu Pro Gly Glu Arg Glu Trp Arg Leu Glu Gln Lys Ala Leu
305                 310                 315                 320

Gln Glu Gly Val Ser Leu Ser Pro Glu Ala Ala Lys Ala Val Gly Leu
                325                 330                 335

<210>  12
<211>  332
<212>  PRT
<213>  Meiothermus ruber

<400>  12

Met Arg Val Pro Tyr Pro Val Leu Lys Gln Ala Val Ser Ser His Phe
1               5                   10                  15

Gln Gly Leu Gly Leu Ala Pro Asp His Ala Glu Ala Phe Thr Glu Val
            20                  25                  30

Ile Leu Glu Ala Glu Leu Glu Gly Asn Leu Gly His Gly Leu Thr Arg
        35                  40                  45

Ile Ala Gln Tyr Thr Ala Gln Leu Gln Ala Gly Gly Leu Asn Pro Arg
    50                  55                  60

Pro Gln Met Arg Leu Glu Arg Thr Lys Pro Gly Val Ala Val Leu His
65                  70                  75                  80

Ala Asp Gly Ala Pro Gly Pro Val Ala Gly Leu Phe Ala Val Gln Ala
                85                  90                  95

Leu Ala Pro Met Ala Arg Glu Gln Gly Ser Ala Ala Leu Ala Val Arg
            100                 105                 110

Gly Ala Gly His Ser Gly Val Leu Ser Ala Tyr Val Gly Arg Leu Ala
        115                 120                 125

Gln Glu Gly Leu Val Ala Leu Ala Phe Ala Asn Thr Pro Pro Ala Ile
    130                 135                 140

Ala Pro Gly Pro Val Leu Gly Thr Asn Pro Ile Ala Leu Gly Ala Pro
145                 150                 155                 160

Ala Glu Pro Gln Pro Val Ile Ile Asp Thr Ser Ile Ser Val Val Ala
                165                 170                 175

Arg Gly Lys Ile Ile Ala Ala Ala Lys Lys Gly Glu Pro Ile Pro Pro
            180                 185                 190

Gly Trp Ala Leu Asp Lys Glu Gly Arg Pro Thr Thr Asp Ala Lys Ala
        195                 200                 205

Ala Leu Glu Gly Ser Leu Leu Pro Ile Gly Glu Gly Lys Gly Phe Ala
    210                 215                 220

Leu Ala Val Leu Val Glu Ile Leu Ala Gly Ala Leu Ala Gly Asp Val
225                 230                 235                 240

Leu Ser Pro Glu Leu Pro Leu Pro Trp Met Pro Pro Ala Gln Ala Ala
                245                 250                 255

Lys Pro Gly Leu Leu Leu Leu Ala Phe Asp Pro Ala Ala Phe Gly Pro
            260                 265                 270

Gly Tyr Arg Gly Arg Val Ala Gln Leu Ile Glu Ala Leu Lys Ala Ala
        275                 280                 285

Gly Gly Arg Ile Pro Gly Ala Arg Arg Ala Ala Leu Arg Glu Lys Ala
    290                 295                 300

Leu Ala Glu Gly Leu Glu Val Asn Gln Thr Leu Gln Ala Glu Leu Gly
305                 310                 315                 320

Thr Leu Gly Val His Leu Gln Gly Gly Gly Thr Arg
                325                 330

<210>  13
<211>  35
<212>  DNA
<213>  Artificial sequence

<220>
<223>  PCR primer

<400>  13
cgtggccaac taggcccagc cagatactcc cgatc                                35

<210>  14
<211>  35
<212>  DNA
<213>  Artificial sequence

<220>
<223>  PCR primer

<400>  14
tgaggcctca ttggccggag cgcaacccac tcact                                35

<210>  15
<211>  35
<212>  DNA

<213>  Artificial sequence

<220>
<223>  PCR primer

<400>  15
ctgggcctag ttggccacgt agaaagccag tccgc                                35


<210>  16
<211>  35
<212>  DNA
<213>  Artificial sequence

<220>
<223>  PCR primer

<400>  16
tccggccaat gaggcctcag aagaactcgt caaga                                35


<210>  17
<211>  83
<212>  DNA
<213>  Artificial sequence

<220>
<223>  PCR primer

<400>  17
gcattaatcc ttggactcct gttgatagat ccagtaatga cctcagaact ccatctggat      60

ttgttcagaa cgctcggttg ccg                                             83


<210>  18
<211>  83
<212>  DNA
<213>  Artificial sequence

<220>
<223>  PCR primer

<400>  18
caccgtgcag tcgatggatc tggattctca ccaataaaaa acgcccggcg gcaaccgagc      60

gttctgaaca aatccagatg gag                                             83


<210>  19
<211>  50
<212>  DNA
<213>  Artificial sequence

<220>
<223>  PCR primer

<400>  19
ttattggtga gaatccagat ccatcgactg cacggtgcac caatgcttct                50


<210>  20
<211>  70

<212>  DNA
<213>  Artificial sequence

<220>
<223>  PCR primer

<400>  20
gcaagcttgg agtgatcatc gtatgcatat gcgtttctcc tccagatccc tgtttcctgt      60

gtgaaattgt                                                              70


<210>  21
<211>  35
<212>  DNA
<213>  Artificial sequence

<220>
<223>  PCR primer

<400>  21
ctcgaattca ctggccgtcg ttttacaacg tcgtg                                  35


<210>  22
<211>  35
<212>  DNA
<213>  Artificial sequence

<220>
<223>  PCR primer

<400>  22
cgcaattgag tttgtagaaa cgcaaaaagg ccatc                                  35


<210>  23
<211>  35
<212>  DNA
<213>  Artificial sequence

<220>
<223>  PCR primer

<400>  23
ttacatatga aacaacaagg catgaatcga gtagc                                  35


<210>  24
<211>  35
<212>  DNA
<213>  Artificial sequence

<220>
<223>  PCR primer

<400>  24
ttagaattct tattttacat catcaaaata acggg                                  35


<210>  25
<211>  35
<212>  DNA

<213>  Artificial sequence

<220>
<223>  PCR primer

<400>  25
ttacatatga aaaacgggag aggaaatcgg gtagc                                35


<210>  26
<211>  35
<212>  DNA
<213>  Artificial sequence

<220>
<223>  PCR primer

<400>  26
ttagaattct tactgagcaa aatagcgcgc caata                                35


<210>  27
<211>  35
<212>  DNA
<213>  Artificial sequence

<220>
<223>  PCR primer

<400>  27
ttacatatga aggtcggcat cgtgggaagc ggcat                                35


<210>  28
<211>  35
<212>  DNA
<213>  Artificial sequence

<220>
<223>  PCR primer

<400>  28
ttagaattcc taaaacccca gggcgaaggc cgcct                                35


<210>  29
<211>  35
<212>  DNA
<213>  Artificial sequence

<220>
<223>  PCR primer

<400>  29
ttacatatga ggtggcgggc ggacttcctc tcggc                                35


<210>  30
<211>  35
<212>  DNA
<213>  Artificial sequence

<220>

<223>  PCR primer

<400>  30
ttagaattct caagcatcgt ccctccaagg cacgc                                35


<210>  31
<211>  35
<212>  DNA
<213>  Artificial sequence

<220>
<223>  PCR primer

<400>  31
ttacatatgc aaggcattcc tgtgcaacaa ctgcg                                35


<210>  32
<211>  35
<212>  DNA
<213>  Artificial sequence

<220>
<223>  PCR primer

<400>  32
ttagaattct taaaggccca ccgctttagc ggcct                                35


<210>  33
<211>  35
<212>  DNA
<213>  Artificial sequence

<220>
<223>  PCR primer

<400>  33
ttacatatga gggttcctta tcccgtactc aagca                                35


<210>  34
<211>  35
<212>  DNA
<213>  Artificial sequence

<220>
<223>  PCR primer

<400>  34
tttgaattct catcttgtcc ctcctccttg tagat                                35

## Description

### Technical Field

The present invention relates to a *Hydrogenophilus* bacterium transformant harboring the ability to produce lactic acid, and to a method for producing lactic acid using the same.

### Background Art

### Production of chemical products using microorganisms

The Paris Agreement, which was adopted in 2015, provides that global emissions of greenhouse gases should be promptly reduced. Under the Agreement, Japan set the goal of reducing her emission of greenhouse gases such as carbon dioxide and methane by 26% compared with year 2013 levels, by the year 2030.

Worldwide, the majority of the production of chemicals depends on petroleum resources, exacerbating the problem of increased greenhouse gas emissions. Accordingly, departure from petroleum dependency is a desirable strategy for the production of chemicals, and research and development of biorefineries that produce green chemicals from biomass is being earnestly carried out in various countries. However, the saccharification of biomass for use as raw materials of microbial fermentation necessitates complex processes, beside being costly.

As part of research geared towards departure from petroleum dependency, gases such as carbon dioxide, methane, and carbon monoxide have attracted attention as more sustainable carbon sources, and techniques for producing valuable chemicals and biofuels using microorganisms that utilize these gases are the subject of intense interest. In particular, carbon fixation of carbon dioxide and efficient utilization of carbon dioxide, a significant contributor to global warming, is highly anticipated.

Biodegradable plastics, which are eventually decomposed to water and carbon dioxide by microorganisms in nature, have attracted attention in light of the problems of sea pollution by plastic garbage, etc. Biodegradable plastics are categorized into bacterial products series, natural products series and chemical synthetic series according to method of manufacture. Polylactic acid (lactic acid resin), of which research and practical realization has proceeded the fastest of all biodegradable plastics, is regarded as an intermediate biodegradable plastic between bacterial products series and chemical synthetic series since its raw material is lactic acid, a product of the glycolysis system, an intravital metabolic pathway. That is, polylactic acid is produced by the purification of lactic acid produced by microbial fermentation and chemical polycondensation. Current polylactic acid production uses biomass as a raw material, the conversion of biomass into saccharides requires complicated steps, as aforementioned, and therefore, current polylactic acid production has a ploblem of a high cost.

Accordingly, a practicable method which is able to produce lactic acid in simpler steps is required. In particular, a practicable method which is able to produce lactic acid by carbon dioxide fixation.

Lactic acid is produced from pyruvic acid, intravital important metabolic product. That is, lactic acid is produced by catalytic activity of lactate dehydrogenase.

As a technology which manufactures lactic acid using a recombinant microorganism, Patent Literature 1 describes a method for producing lactic acid using a transformant obtained by introducing the lactate dehydrogenase gene of *Lactobacillus helvetics* or *Bacillus megaterium* into a yeast strain.

Patent Literature 2 describes a method for producing lactic acid using a transformant obtained by introducing *Lactobacillus pentosus* LDH gene as a lactate dehydrogenase gene into *Schizosaccharomyces pombe.*

Patent Literature 3 describes a method for producing lactic acid using a transformant obtained by introducing *Thermoanaerobacter pseudethanolicus* ldh gene as a lactate dehydrogenase gene into *Moorella thermoacetica.*

Patent Literature 4 describes a method for producing lactic acid using a transformant obtained by introducing *Lactobacillus delbrueckii* hdhD gene or ldhA gene as a lactate dehydrogenase gene into *Geobacillus thermoglucosidans.*

Non Patent Literature 1 describes a method for producing lactic acid using a transformant obtained by introducing the lactate dehydrogenase gene of *Lactobacillus casei* into *Escherichia coli.*

However, all these methods are methods for producing lactic acid using sugar as a carbon source, and not methods for producing lactic acid using carbon dioxide as a carbon source.

Non Patent Literature 2 describes a method for producing lactic acid using a transformant obtained by introducing the lactate dehydrogenase gene of *Bacillus subtilis* into *Synechocystis sp.* PCC6803 strain. This method is for producing lactic acid using Cyanobacterium, which is a photosynthetic bacterium, as a host and using sodium hydrogen carbonate as a carbon source.

Cyanobacteria have a higher carbon fixation ability of carbon dioxide as compared to that of plants. However, the method of using Cyanobacterium as a host has not been put into practical use as an industrial method for producing lactic acid since carbon dioxide fixation ability of Cyanobacteria is insufficient.

Patent Literature 5 describes a method for producing lactic acid using a transformant obtained by introducing *Thermus thermophilus* ldhA gene as a lactate dehydrogenase gene into *Hydrogenobacter thermophilus.*

*Hydrogenobacter thermophilus* is a hydrogen oxidizing bacterium which grows twofold in 1.5 hours. However, to apply current is necessary in order to produce sufficient amounts of lactic acid, and therefore, the method using *Hydrogenobacter thermophilus* as a host has not been put into practical use as an industrial method for producing lactic acid.

### Citation List

### Patent Literatures

[Patent Literature 1] JP2005-528106A
[Patent Literature 2] JP2014/030655A1
[Patent Literature 3] JP2015-023854A
[Patent Literature 4] JP2017-523778A
[Patent Literature 5] JP2017-093465A

### Non Patent Literatures

[Non Patent Literature 1] Homofermentative production of D- or L-lactate in metabolically engineered Escherichia coli RR1. Chang DE, Jung HC, Rhee JS, Pan JG. Appl. Environ. Microbiol. (1999) 65:1384-1389
[Non Patent Literature 2] Engineering a cyanobacterial cell factory for production of lactic acid. Angermayr SA, Paszota M, Hellingwerf KJ. Appl. Environ. Microbiol. (2012) 78:7098-7106

### Summary of Invention

### Technical Problem

The objective of the present invention is to provide a transformant of a *Hydrogenophilus* bacterium that is capable of efficiently producing lactic acid utilizing carbon dioxide as a sole carbon source, and a method for efficiently producing lactic acid using this transformant.

### Solution to Problem

*Hydrogenophilus* bacteria are hydrogen oxidizing bacteria which grow by producing organic substances from carbon dioxide by utilizing hydrogen energy. The growth rate of hydrogen-oxidizing bacteria is generally extremely slow, however, the growth rate of *Hydrogenophilus* bacteria is fast, and their carbon fixation ability of carbon dioxide is remarkably higher than that of plants and photosynthetic bacteria.

*Hydrogenophilus* bacteria do not have a lactate dehydrogenase gene and a malate/lactate dehydrogenase gene, which are known to encode an enzyme catalyzing the reaction of producing lactic acid from pyrubic acid. In order to provide the bacteria with an ability to produce lactic acid at an industrial scale, there is a need to introduce genes of enzymes that catalyze the reaction of producing lactic acid.

The inventors of the present invention have found that when a heterologous gene is introduced into *Hydrogenophilus* bacteria using a vector that functions within the *Hydrogenophilus* bacteria, a functioning protein often is not produced or is insufficiently produced. Genes which bring about high activity within bacteria other than the genus *Hydrogenophilus* often do not, or insufficiently, bring about activity.

Faced with such a situation, the inventors of the present invention have found that when a lactate dehydrogenase gene and/or a gene encoding a malate/lactate dehydrogenase which has lactate dehydrogenase activity is/are introduced into *Hydrogenophilus* bacteria, the gene(s) function(s) and bring(s) about high activity within the *Hydrogenophilus* bacteria.

The inventors of the present invention have also found that a transformant obtained by introducing a lactate dehydrogenase gene and/or a malate/lactate dehydrogenase gene into a *Hydrogenophilus* bacterium, efficiently produces lactic acid using carbon dioxide as a sole carbon source.

Further, the inventors of the present invention have found that ldh gene of *Parageobacillus thermoglucosidasius, Geobacillus kaustophilus* or *Thermus thermophilus* of the lactate dehydrogenase genes and mldh gene of *Thermus thermophilus* and mldh-1 and mldh-2 genes of *Meiothermus ruber* of the malate/lactate dehydrogenase genes bring about higher enzymatic activity expression especially in *Hydrogenophilus* bacteria.

The present invention has been completed based on the above-mentioned findings, and provides the following transformants and methods for producing lactic acid.
Aspect 1. A transformant obtained by introducing (a) a lactate dehydrogenase gene and/or (b) a malate/lactate dehydrogenase gene into a *Hydrogenophilus* bacterium.
Aspect 2. The transformant according to aspect 1, wherein (a) the lactate dehydrogenase gene is the following DNA (a1), (a2), (a3), (a4), (a5) or (a6):
   (a1) DNA which consists of a base sequence of SEQ ID NO: 1, 2 or 3;
   (a2) DNA which consists of a base sequence having 90% or more identity to SEQ ID NO: 1, 2 or 3, the DNA encoding a polypeptide having lactate dehydrogenase activity;
   (a3) DNA which hybridizes with a DNA consisting of a base sequence complementary to SEQ ID NO: 1, 2 or 3 under stringent conditions, and which encodes a polypeptide having lactate dehydrogenase activity;
   (a4) DNA which encodes a polypeptide consisting of an amino acid sequence of SEQ ID NO: 4, 5 or 6;
   (a5) DNA which encodes a polypeptide consisting of an amino acid sequence having 90% or more identity to SEQ ID NO: 4, 5 or 6, the polypeptide having lactate dehydrogenase activity; (a6) DNA which encodes a polypeptide consisting of an amino acid sequence having a deletion, substitution, or addition of one or a plurality of amino acids in an amino acid sequence of SEQ ID NO: 4, 5 or 6, the polypeptide having lactate dehydrogenase activity.
Aspect 3. The transformant according to aspect 1 or 2, wherein (b) the malate/lactate dehydrogenase gene is the following DNA (b1), (b2), (b3), (b4), (b5) or (b6):
   (b1) DNA which consists of a base sequence of SEQ ID NO: 7, 8 or 9;
   (b2) DNA which consists of a base sequence having 90% or more identity to SEQ ID NO: 7, 8 or 9, the DNA encoding a polypeptide having lactate dehydrogenase activity;
   (b3) DNA which hybridizes with a DNA consisting of a base sequence complementary to SEQ ID NO: 7, 8 or 9 under stringent conditions, and which encodes a polypeptide having lactate dehydrogenase activity;
   (b4) DNA which encodes a polypeptide consisting of an amino acid sequence of SEQ ID NO: 10, 11 or 12;
   (b5) DNA which encodes a polypeptide consisting of an amino acid sequence having 90% or more identity to SEQ ID NO: 10, 11 or 12, the polypeptide having lactate dehydrogenase activity;
   (b6) DNA which encodes a polypeptide consisting of an amino acid sequence having a deletion, substitution, or addition of one or a plurality of amino acids in the amino acid sequence of SEQ ID NO: 10, 11 or 12, the polypeptide having lactate dehydrogenase activity.
Aspect 4. The transformant according to any one of aspects 1-3, wherein the *Hydrogenophilus* bacterium is *Hydrogenophilus thermoluteolus.*
Aspect 5. A method for producing lactic acid comprising a step of culturing the transformant according to any one of aspects 1-4 through use carbon dioxide as a substantially sole carbon source.

### Advantageous Effects of Invention

Measures to counter the increase in atmospheric carbon dioxide entail reduction of carbon dioxide emissions and fixation of emitted carbon dioxide. In order to reduce carbon dioxide emissions, solar, wind, geothermal, and similar energies are utilized in place of fossil energy. However, the utilization of such energies is not yet extensive enough to repress the buildup of atmospheric carbon dioxide. Consequently, there is need to enhance atmospheric carbon fixation or recycling of emitted carbon dioxide.

Carbon fixation of carbon dioxide can occur physically or chemically, but fixation utilizing living cells, avails organic substances that can consequently be utilized as food, feed, and fuel. In so doing, carbon dioxide itself becomes a resource that can be directly converted into valuable chemical products. Accordingly, the twin problems of global warming due to increased atmospheric carbon dioxide and scarcity of food, feed, and fuel can be solved. Further, in-demand chemical products can be produced while suppressing global warming attributed to increased carbon dioxide emissions.

Biodegradable plastics of chemical products attract attention for their environmental benefits. Biodegradable plastics produced by fixation of carbon dioxide are decomposed to water and carbon dioxide by microorganisms in the environment. That is, biodegradable plastics are carbon-neutral, and are able to solve global warming attributed to increased carbon dioxide emissions, difficulty in securing plastic products necessary for life, and environmental problems such as sea pollution, together.

Hydrogen-oxidizing bacteria can grow by utilizing the chemical energy generated by the reaction of hydrogen with oxygen and by using carbon dioxide as a sole carbon source. Since hydrogen-oxidizing bacteria can produce chemical products from a mixture of oxygen, hydrogen, and carbon dioxide gases as raw material, the cells can efficiently assimilate carbon from carbon dioxide and be cultured in a simple culture medium. Growth of typical hydrogen-oxidizing bacteria is generally slow, but that of *Hydrogenophilus* bacteria is exceptionally high. The Journal of Mitsubishi Research Institute No.34 1999 describes *Hydrogenophilus* bacteria as follows: "Their proliferative capacity is so high that their carbon fixation ability of carbon dioxide cannot be compared with that of plants, which truly indicates the high carbon dioxide fixation ability of microorganisms".

When a heterologous gene is introduced into *Hydrogenophilus* bacteria using a vector that functions within the *Hydrogenophilus* bacteria, a functioning protein is often not produced. Regardless, according to the present invention, by introducing lactate dehydrogenase gene and/or malate/lactate dehydrogenase gene into of *Hydrogenophilus* bacteria, the genes functioned within the *Hydrogenophilus* bacteria, and lactic acid could be produced.

As described above, *Hydrogenophilus* bacteria have a atypically remarkable carbon fixation ability of carbon dioxide among organisms having carbon dioxide fixation ability, and therefore, by using the transformant of the present invention, carbon derived from carbon dioxide can be fixed and lactic acid can be produced at an industrial level. Since lactic acid is used as a raw material for producing polylactic acid, which is a typical biodegradable plastic, the present invention has opened the way to producing polylactic acid industrially.

### Mode for Carrying Out the Invention

The present invention is described in detail below:

### (1) Transformant having lactic acid producing ability

The present invention encompasses a transformant obtained by introducing lactate dehydrogenase gene and/or malate/lactate dehydrogenase gene into a host bacterium of *Hydrogenophilus.* In other words, this transformant possesses exogenous lactate dehydrogenase gene and/or malate/lactate dehydrogenase gene. Malate/lactate dehydrogenase is an enzyme having the activity of lactate dehydrogenase.

Lactate dehydrogenase gene or malate/lactate dehydrogenase gene can be introduced, alternatively, lactate dehydrogenase gene and malate/lactate dehydrogenase gene can be introduced. Further, two or more kinds of lactate dehydrogenase genes and/or two or more kinds of malate/lactate dehydrogenase genes can be introduced.

*Hydrogenophilus* bacteria do not produce lactic acid in an amount that can be utilized industrially. When a lactate dehydrogenase gene and/or malate/lactate dehydrogenase gene of a heterogenous microorganism is introduced into *Hydrogenophilus* bacteria, the gene(s) function(s) within the *Hydrogenophilus* bacteria, and a highly active lactate dehydrogenase and/or malate/lactate dehydrogenase is/are produced, and therefore, the obtained transformants efficiently produce lactic acid using carbon dioxide as a sole carbon source.

### Transgene

Examples of the lactate dehydrogenase gene include *Parageobacillus thermoglucosidasius* ldh gene, *Geobacillus kaustophilus* ldh gene and *Thermus thermophilus* ldh gene, which are preferable in that they have good lactic acid production efficiency. The base sequence of *Parageobacillus thermoglucosidasius* ldh gene is SEQ ID NO: 1, the base sequence of *Geobacillus kaustophilus* ldh gene is SEQ ID NO: 2 and base sequence of *Thermus thermophilus* ldh gene is SEQ ID NO: 3.

DNA which consists of a base sequence having 90% or more, particularly 95% or more, more particularly 98% or more, furthermore particularly 99% or more identity to SEQ ID NO: 1, 2 or 3, the DNA encoding a polypeptide having lactate dehydrogenase activity, can also be used preferably.

In the present invention, the identities of base sequences were calculated using GENETYX ver.17 (made by GENETYX Corporation).

DNA which hybridizes with a DNA consisting of a base sequence complementary to SEQ ID NO: 1, 2 or 3 under stringent conditions, the DNA encoding a polypeptide having lactate dehydrogenase activity, can also be used preferably.

In the present invention, "stringent conditions" means hybridization with 6xSSC solution at temperatures from 50 to 60°C for 16 hours, followed by washing with 0.1xSSC solution.

In addition, DNA which encodes a polypeptide consisting of an amino acid sequence of SEQ ID NO: 4, 5 or 6 is also used preferably. SEQ ID NO: 4 is the amino acid sequence of *Parageobacillus thermoglucosidasius* lactate dehydrogenase, SEQ ID NO: 5 is the amino acid sequence of *Geobacillus kaustophilus* lactate dehydrogenase, and SEQ ID NO: 6 is the amino acid sequence of *Thermus thermophilus* lactate dehydrogenase.

Further, DNA which encodes a polypeptide consisting of an amino acid sequence having 90% or more, preferably 95% or more, more preferably 98% or more, even more preferably 99% or more identity to SEQ ID NO: 4, 5 or 6, the polypeptide having lactate dehydrogenase activity can also be used.

In the present invention, the identities of amino acid sequences were calculated using GENETYX ver.17 (made by GENETYX Corporation).

DNA which encodes a polypeptide consisting of an amino acid sequence having a deletion, substitution, or addition of one or a plurality of amino acids in the amino acid sequence of SEQ ID NO: 4, 5 or 6, the polypeptide having lactate dehydrogenase activity can also be used preferably.

In the present invention, examples of plurality include 1 to 5, in particular 1 to 3, in particular 1 to 2, and particularly 1.

Examples of the malate/lactate dehydrogenase gene include *Thermus thermophilus* mldh gene and *Meiothermus ruber* mldh-1 and mldh-2 genes, which are preferable in that they have good lactic acid production efficiency. The base sequence of *Thermus thermophilus* mldh gene is SEQ ID NO: 7, the base sequence of *Meiothermus ruber* mldh-1 gene is SEQ ID NO: 8 and the base sequence of *Meiothermus ruber* mldh-2 gene is SEQ ID NO: 9.

DNA which consists of a base sequence having 90% or more, particularly 95% or more, more particularly 98% or more, further more particularly 99% or more identity to SEQ ID NO: 7, 8 or 9, the DNA encoding a polypeptide having lactate dehydrogenase activity, and DNA which hybridizes with a DNA consisting of a base sequence complementary to SEQ ID NO: 7, 8 or 9 under stringent conditions, the DNA encoding a polypeptide having lactate dehydrogenase activity, can also be used preferably.

In addition, DNA which encodes a polypeptide consisting of an amino acid sequence of SEQ ID NO: 10, 11 or 12 is also used preferably. SEQ ID NO: 10 is the amino acid sequence which is encoded by *Thermus thermophilus* malate/lactate dehydrogenase (Mldh) gene, SEQ ID NO: 11 is the amino acid sequence which is encoded by *Meiothermus ruber* malate/lactate dehydrogenase (Mldh-1) gene, and SEQ ID NO: 12 is the amino acid sequence which *is encoded by Meiothermus ruber* malate/lactate dehydrogenase (Mldh-2) gene.

Further, DNA which encodes a polypeptide consisting of an amino acid sequence having 90% or more, particularly 95% or more, more particularly 98% or more, further more particularly 99% or more identity to SEQ ID NO: 10, 11 or 12, the polypeptide having lactate dehydrogenase activity, and DNA which encodes a polypeptide consisting of an amino acid sequence having a deletion, substitution, or addition of one or a plurality of amino acids in the amino acid sequence of SEQ ID NO: 10, 11 or 12, the polypeptide having lactate dehydrogenase activity can also be used preferably.

In the present invention, in order to verify that a polypeptide to be tested has a lactate dehydrogenase activity, the polypeptide is reacted with pyruvic acid under the coexistence of NADH, and decrease in absorbance at 340nm is detected. Lactate dehydrogenase produces lactic acid from pyruvic acid. Lactate dehydrogenase consumes NADH when lactic acid is produced from pyruvic acid, and thus decrease in the amount of NADH is detected using decrease in absorbance at 340nm as an index. Specifically, the method described in item "Examples" is carried out. If the polypeptide to be tested reduces absorbance at 340nm even by a slight degree, the polypeptide is determined to have lactate dehydrogenase activity.

### (2) Methods for producing transformants

Next, methods for obtaining transformants by introducing the above-described genes for the production of lactic acid into *Hydrogenophilus* bacteria are described.

### Host

Examples of *Hydrogenophilus* bacteria include *Hydrogenophilus thermoluteolus, Hydrogenophilus halorhabdus, Hydrogenophilus denitrificans, Hydrogenophilus hirschii, Hydrogenophilus islandicus,* and strain Mar3 of the genus *Hydrogenophilus* (*Hydrogenophilus* sp. Mar3). In particular, *Hydrogenophilus thermoluteolus* is preferable because its superior growth rate enables top-level carbon fixation from cardon dioxide among carbon dioxide fixing microorganisms.

*Hydrogenophilus* bacteria have been easily isolated from diverse regions everywhere on the earth. A preferable strain of *Hydrogenophilus thermoluteolus* is strain TH-1 (NBRC 14978). *Hydrogenophilus thermoluteolus* strain TH-1 (NBRC 14978) exhibits comparatively rapid growth rate among carbon dioxide fixing microorganisms (Agricultural and Biological Chemistry, 41, 685-690 (1977)). *Hydrogenophilus thermoluteolus* strain NBRC 14978 is internationally deposited under the Budapest Treaty, and is thus available to the general public.

### Transformation

Plasmid vectors for introducing the above-described DNAs into a host should contain a DNA which controls the autonomous replication function within *Hydrogenophilus* bacteria, and examples include broad-host-range vectors pRK415 (GenBank: EF437940.1), pBHR1 (GenBank: Y14439.1), pMMB67EH (ATCC 37622), pCAR1 (NCBI Reference Sequence: NC_004444.1), pC194 (NCBI Reference Sequence: NC_002013.1), pK18mobsacB (GenBank: FJ437239.1), pUB110 (NCBI Reference Sequence: NC_001384.1), and the like.

Examples of preferable promoters include tac promoter, lac promoter, trc promoter, or each of promoters OXB1 and OXB11 to OXB20 from Oxford Genetics Ltd. Examples of preferable terminators include the T1T2 terminator of *Escherichia coli* rRNA operon rrnB, the t0 transcription terminator of bacteriophage λ, and the like.

Transformation can be carried out by publicly known methods such as calcium chloride method, calcium phosphate method, DEAE-dextran transfection method, and electric pulse method.

*Hydrogenophilus* bacteria grow under autotrophic conditions. However, since they can grow under heterotrophic conditions as well, the culture medium which is used to culture a host or *Hydrogenophilus* bacterium recombinant can either be an inorganic culture medium or an organic culture medium. An organic culture medium comprising sugar, organic acids, amino acid, and the like can be used. The pH of the culture medium can be adjusted to approximately 6.2 to 8.

In any of the cases, culture can be carried out while supplying a mixture of gases containing hydrogen, oxygen, and carbon dioxide, and preferably a mixture of gases consisting of hydrogen, oxygen, and carbon dioxide. When using an organic culture medium, a mixture of gases containing hydrogen, oxygen, and carbon dioxide, for example air, can be used for aeration. When carbon dioxide gas is not supplied, a culture medium containing a carbonate as a carbon source can be used. Mixed gases can be entrapped within or continuously supplied into an airtight culture container, and can be dissolved into the culture medium by means of shaking culture. Alternatively, the culture container can be an airtight or open type, and mixed gases can be dissolved into the culture medium by bubbling.

The volume ratio of hydrogen, oxygen, and carbon dioxide within the supplied gas (hydrogen: oxygen: carbon dioxide) is preferably 1.75 to 7.5:1:0.25 to 3, more preferably 5 to 7.5:1:1 to 2, and furthermore preferably 6.25 to 7.5:1:1.5. *Hydrogenophilus* bacteria are thermophilic bacteria, and thus the culture temperature is preferably 35 to 55°C, more preferably 37 to 52°C, and even more preferably 50 to 52°C.

### (3) Method for producing lactic acid

When producing lactic using the transformant of a *Hydrogenophilus* bacterium described above, the transformant can be cultured using an inorganic or organic culture medium while supplying a mixture of gases containing hydrogen, oxygen, and carbon dioxide.

The supplied gas is preferably a mixture of gases consisting of hydrogen, oxygen, and carbon dioxide. However, different kinds of gases can be mixed within, to the extent that lactic acid can be produced efficiently.

*Hydrogenophilus* bacteria can grow using hydrogen as a source of energy and using carbon dioxide as a sole carbon source, and thus, carbon dioxide can be fixed efficiently particularly by producing the above-described compounds by using substantially only carbon dioxide (in particular, by using only carbon dioxide) as a carbon source. Therefore, using an inorganic culture medium that does not contain carbon sources such as organic substances and carbonates, namely, carrying out culture using substantially only carbon dioxide (in particular, using only carbon dioxide) as a carbon source is preferable. "Using substantially only carbon dioxide as a carbon source" encompasses cases in which an unavoidable amount of other carbon sources is mixed within. Furthermore, a culture medium containing organic substances such as sugar, organic acids, and amino acids, as well as carbonates, can also be used without supplying carbon dioxide.

The pH of the culture medium is preferably 6.2 to 8, more preferably 6.4 to 7.4, and furthermore preferably 6.6 to 7. When the pH is within this range, bacteria grow well and mixed gas dissolves well into the culture medium, and lactic acid can be produced efficiently.

When batch culture is utilized, mixed gases can be entrapped within an airtight culture container and static culture or shaking culture can be carried out. When continuous culture is utilized, mixed gases can be continuously supplied into an airtight culture container and shaking culture can be carried out, or the transformant can be cultured using an airtight culture container while introducing mixed gases into the culture medium by bubbling. Shaking culture is preferable in that better dissolution of mixed gases into the culture medium can be achieved.

The volume ratio of hydrogen, oxygen, and carbon dioxide (hydrogen: oxygen: carbon dioxide) in the supplied gas mixture is preferably 1.75 to 7.5:1:0.25 to 3, more preferably 5 to 7.5:1:1 to 2, and even more preferably 6.25 to 7.5:1:1.5. When the volume ratio is within this range, bacteria grow well, and the target compound can be produced efficiently.

The supply rate of mixed gases or raw material gases can be 10.5 to 60 L/hour, in particular 10.5 to 40 L/hour, in particular 10.5 to 21 L/hour, per 1 L of culture medium. When the supply rate is within this range, transformants grow well and the target compound can be produced efficiently, and the amount of wasted mixed gases can be reduced.

The culture temperature is preferably 35 to 55°C, more preferably 37 to 52°C, and even more preferably 50 to 52°C. When the temperature is within this range, transformants grow well, and lactic acid can be produced efficiently.

The target compound lactic acid is produced in the reaction solution by culturing in the above-described manner. Collecting the reaction solution will enable the recovery of lactic acid, however, lactic acid can furthermore be separated from the reaction solution by publicly known methods. Such publicly known methods include precipitation method, fractional distillation and electrodialysis.

### Examples

### (1) Construction of a plasmid vector

The method for constructing a plasmid vector that was commonly used to introduce genes for conferring lactic acid producing ability is described below.

First, a broad-host-range vector pRK415 (GenBank: EF437940.1) (Gene, 70, 191-197 (1998)) was used as a template and PCR was performed. In order to amplify the DNA fragment of the plasmid region excluding a tetracycline gene region, a primer pair described below was synthesized and used. PCR was performed according to a conventional method using "DNA thermal cycler" manufactured by Life Technologies Inc., and using KOD FX Neo (manufactured by Toyobo Co., Ltd.) as a reaction reagent.
Primers for the amplification of pRK415 plasmid sequence
(a-1) 5'-CGTGGCCAACTAGGCCCAGCCAGATACTCCCGATC-3' (SEQ ID NO: 13)
(b-1) 5'-TGAGGCCTCATTGGCCGGAGCGCAACCCACTCACT-3' (SEQ ID NO: 14)
A SfiI restriction site has been added to primers (a-1) and (b-1) .

Plasmid pK18mobsacB (GenBank: FJ437239.1) (Gene, 145, 69-73 (1994)), which contains a neomycin/kanamycin resistance gene (hereinafter, the gene may be referred to as "nptII"), was used as a template and PCR was performed according to a conventional method. In the PCR, a primer pair described below was synthesized and used in order to amplify the DNA fragment containing the nptII gene sequence. PCR was performed according to a conventional method using "DNA thermal cycler" manufactured by Life Technologies Inc., and using KOD FX Neo (manufactured by Toyobo Co., Ltd.) as a reaction reagent.
Primers for the amplification of nptII gene sequence
(a-2) 5'-ctgGGCCTAGTTGGCCacgtagaaagccagtccgc-3' (SEQ ID NO: 15)
(b-2) 5'-tccGGCCAATGAGGCCtcagaagaactcgtcaaga-3' (SEQ ID NO: 16)
A SfiI restriction site has been added to primers (a-2) and (b-2) .

The reaction solutions that were produced by each of the above-described PCR were subjected to electrophoresis using a 1% agarose gel, and as a result, a DNA fragment of approximately 8.7-kb was detected when pRK415 plasmid was used as a template, and a DNA fragment of approximately 1.1-kb was detected when nptII gene was used as a template.

Thus prepared DNA fragments were each cleaved by restriction enzyme SfiI, and reacted with a T4 DNA Ligase (manufactured by Takara Bio Inc.) to obtain a ligation solution. The obtained ligation solution was used to transform *Escherichia coli* JM109 by calcium chloride method (Journal of Molecular Biology, 53, 159-162 (1970)), and the transformants were applied onto LB agar media containing 50 µg/mL kanamycin. Viable strains on the culture media were cultured in a liquid culture medium by a conventional method, and plasmid DNA was extracted from the obtained culture solution. This plasmid DNA was cleaved by using restriction enzyme SfiI, and the inserted fragment was confirmed. As a result, a DNA fragment of the nptII gene sequence which was approximately 1.1-kb was observed in addition to DNA fragments of approximately 2.0-kb, 3.0-kb and 3.7-kb, which were derived from the pRK415 plasmid.

The constructed plasmid was named pCYK01.

### (2) Construction of cloning vector used for gene expression

### (2-1) Preparation of DNA fragment of λ t0 terminator sequence

A primer pair described below was synthesized and used in PCR in order to prepare a DNA having λ t0 terminator sequence. PCR was performed using "DNA thermal cycler" manufactured by Life Technologies Inc., and using KOD FX Neo (manufactured by Toyobo Co., Ltd.) as a reaction reagent. No template DNA was included since extension was carried out using each primer as the other's template.
Primers for the preparation of λ t0 terminator sequence
(a-3) 5'-GCATTAATccttggactcctgttgatagatccagtaatgacctcagaactccatctggattt gttcagaacgctcggttgccg -3' (SEQ ID NO: 17)
(b-3) 5'-caccgtgcagtcgatgGATctggattctcaccaataaaaaacgcccggcggcaaccgagcgt tctgaacaaatccagatggag -3' (SEQ ID NO: 18)
The base sequences of the 3' ends of primers (a-3) and (b-3) are complementary to each other.

The produced reaction solution was subjected to electrophoresis using a 1% agarose gel, and as a result, a DNA fragment of approximately 0.13-kb, which corresponds to the λ t0 terminator sequence, was detected.

### (2-2) Preparation of a DNA fragment of tac promoter sequence

PCR was performed using plasmid pMAL-c5X (manufactured by New England Biolabs Inc.) containing a tac promoter, as a template. In the PCR, a primer pair described below was synthesized and used in order to amplify tac promoter sequence. PCR was performed according to a conventional method using "DNA thermal cycler" manufactured by Life Technologies Inc., and using KOD FX Neo (manufactured by Toyobo Co., Ltd.) as a reaction reagent.
Primers for the amplification of tac promoter sequence
(a-4) 5'-TTATTGGTGAGAATCCAGATCCATCGACTGCACGGTGCACCAATGCTTCT-3' (SEQ ID NO: 19)

(b-4) 5′-
gcaagcttggagtgatcatcgtATGCATATGCGTTTCTCCTCCAGATCCctgtttcctgtgt
gaaattgt
-3′ (SEQ ID NO: 20)

The produced reaction solution was subjected to electrophoresis using a 1% agarose gel, and as a result, a DNA fragment of approximately 0.3-kb, which corresponds to tac promoter sequence, was detected.

### (2-3) Introduction of λ t0 terminator and tac promoter sequences

The DNA fragments that were prepared in the above-described (2-1) and (2-2) were cut out from the agarose gel, and DNA was recovered from the gel by freezing and melting the gel. The recovered DNA fragments corresponding to λ t0 terminator sequence and the tac promoter sequence were mixed and used as templates, and overlap extension PCR was performed. In the overlap extension PCR, a combination of the above-described primers (a-3) and (b-4) was used in order to prepare a DNA in which the tac promoter is linked downstream of λt0 terminator. The base sequences of the 5' ends of the primers (b-3) and (a-4), which were used in amplifying the template DNA fragments, are complementary with each other. PshBI and HindIII restriction sites have been added to primers (a-3) and (b-4), respectively.

The produced reaction solution was subjected to electrophoresis using a 1% agarose gel, and as a result, a DNA fragment of approximately 0.4-kb, which corresponds to the DNA in which the tac promoter is linked downstream of λ t0 terminator, was detected.

The approximately 0.4-kb DNA fragment that was amplified by PCR, in which the tac promoter is linked downstream of the λ t0 terminator, and the above-mentioned approximately 9.8-kb DNA fragment of cloning vector pCYK01, were cleaved by the restriction enzymes PshBI and HindIII. The cleaved DNA fragments were linked to each other using a T4 DNA Ligase (manufactured by Takara Bio Inc.).

The obtained ligation solution was used to transform *Escherichia coli* JM109 by calcium chloride method, and the transformants were applied onto LB agar media containing 50 µg/mL kanamycin. Viable strains on the culture media were cultured in a liquid culture medium by a conventional method, and plasmid DNA was extracted from the obtained culture solution. This plasmid DNA was cleaved by using restriction enzymes PshBI and HindIII, and the inserted fragment was confirmed. As a result, a DNA fragment of approximately 0.4-kb, in which tac promoter is linked downstream of λ t0 terminator, was observed in addition to a DNA fragment of approximately 9.6-kb from plasmid pCYK01.

### (2-4) Introduction of rrnB T1T2 bidirectional terminator (hereinafter, may be referred to as "rrnB terminator")

PCR was performed using plasmid pMAL-c5X (manufactured by New England Biolabs Inc.) containing rrnB terminator sequence as a template. In the PCR, a primer pair described below was synthesized and used in order to amplify rrnB terminator sequence. PCR was performed according to a conventional method using "DNA thermal cycler" manufactured by Life Technologies Inc., and using KOD FX Neo (manufactured by Toyobo Co., Ltd.) as a reaction reagent.
Primers for the amplification of rrnB terminator sequence
(a-5) 5'-ctcgaattcactggccgtcgttttacaacgtcgtg-3' (SEQ ID NO: 21)
(b-5) 5'-CGCAATTGAGTTTGTAGAAACGCAAAAAGGCCATC-3' (SEQ ID NO: 22)
EcoRI and MunI restriction sites have been added to primers (a-5) and (b-5), respectively.

The produced reaction solution was subjected to electrophoresis using a 1% agarose gel, and as a result, a DNA fragment of approximately 0.6-kb, which corresponds to rrnB terminator sequence, was detected.

The approximately 0.6-kb DNA fragment containing rrnB terminator sequence, which was amplified by the above-described PCR, was cleaved by restriction enzymes EcoRI and MunI, and the approximately 10.0-kb DNA fragment of the plasmid that was constructed in the above-described (2-3) was cleaved using restriction enzyme EcoRI. The cleaved DNA fragments were linked to each other using a T4 DNA Ligase (manufactured by Takara Bio Inc.).

The obtained ligation solution was used to transform *Escherichia coli* JM109 by calcium chloride method, and the obtained transformants were applied onto LB agar media containing 50 µg/mL kanamycin. Viable strains on the culture media were cultured in a liquid culture medium by a conventional method, and plasmid DNA was extracted from the obtained culture solution. This plasmid was cleaved by using restriction enzymes EcoRI and MunI, and the inserted fragment was confirmed. As a result, a DNA fragment of approximately 0.6-kb which corresponds to rrnB terminator sequence was observed in addition to a DNA fragment of approximately 10.0-kb from the above-described plasmid of (2-3).

The constructed cloning vector for gene expression was named pCYK21.

### (3) Transformant capable of producing lactic acid

### (3-1) Cloning of lactate dehydrogenase gene

Genomic DNAs were extracted from *Parageobacillus thermoglucosidasius* NBRC 107763, *Geobacillus kaustophilus* NBRC 102445, and *Meiothermus ruber* NBRC 106122 according to a conventional method. Genomic DNA of *Thermus thermophilus* HB8 strain (ATCC 27634) was purchased from Takara Bio Inc.

The four genomic DNAs described above were each used as templates to amplify a DNA fragment containing lactate dehydrogenase ldh gene of each of *Parageobacillus thermoglucosidasius, Geobacillus kaustophilus* and *Thermus thermophilus* and a DNA fragment containing malate/lactate dehydrogenase mldh gene of each of *Thermus thermophilus* and *Meiothermus ruber,* respectively, by PCR method. The following primers were used for PCR. PCR was performed according to a conventional method using "DNA thermal cycler" manufactured by Life Technologies Inc., and using KOD FX Neo (manufactured by Toyobo Co., Ltd.) as a reaction reagent.

Primers for the amplification of *Parageobacillus thermoglucosidasius* ldh gene
(a-6) 5'-TTACATATGAAACAACAAGGCATGAATCGAGTAGC-3' (SEQ ID NO: 23)
(b-6) 5'-TTAGAATTCTTATTTTACATCATCAAAATAACGGG-3' (SEQ ID NO: 24)
An NdeI restriction site has been added to primer (a-6), and an EcoRI restriction site has been added to primer (b-6).

Primers for the amplification of *Geobacillus kaustophilus* ldh gene
(a-7) 5'-TTACATATGAAAAACGGGAGAGGAAATCGGGTAGC-3' (SEQ ID NO: 25)
(b-7) 5'-TTAGAATTCTTACTGAGCAAAATAGCGCGCCAATA-3' (SEQ ID NO: 26)
An NdeI restriction site has been added to primer (a-7), and an EcoRI restriction site has been added to primer (b-7).

Primers for the amplification of *Thermus thermophilus* ldh gene
(a-8) 5'-TTACATATGAAGGTCGGCATCGTGGGAAGCGGCAT-3' (SEQ ID NO: 27)
(b-8) 5'-TTAGAATTCCTAAAACCCCAGGGCGAAGGCCGCCT-3' (SEQ ID NO: 28)
An NdeI restriction site has been added to primer (a-8), and an EcoRI restriction site has been added to primer (b-8).

Primers for the amplification of *Thermus thermophilus* mldh gene
(a-9) 5'-TTACATATGAGGTGGCGGGCGGACTTCCTCTCGGC-3' (SEQ ID NO: 29)
(b-9) 5'-TTAGAATTCTCAAGCATCGTCCCTCCAAGGCACGC-3' (SEQ ID NO: 30)
An NdeI restriction site has been added to primer (a-9), and an EcoRI restriction site has been added to primer (b-9).

Primers for the amplification of *Meiothermus ruber* mldh-1 gene
(a-10) 5'-TTACATATGCAAGGCATTCCTGTGCAACAACTGCG-3' (SEQ ID NO: 31)
(b-10) 5'-TTAGAATTCTTAAAGGCCCACCGCTTTAGCGGCCT-3' (SEQ ID NO: 32)
An NdeI restriction site has been added to primer (a-10), and an EcoRI restriction site has been added to primer (b-10).

Primers for the amplification of *Meiothermus ruber* mldh-2 gene
(a-11) 5'-TTACATATGAGGGTTCCTTATCCCGTACTCAAGCA-3' (SEQ ID NO: 33)
(b-11) 5'-TTTGAATTCTCATCTTGTCCCTCCTCCTTGTAGAT-3' (SEQ ID NO: 34)
An NdeI restriction site has been added to primer (a-11), and an EcoRI restriction site has been added to primer (b-11).

The produced reaction solutions were subjected to electrophoresis using a 1% agarose gel, and DNA fragments of approximately 1.0-kb were detected with regard to each of *Parageobacillus thermoglucosidasius ldh* gene, *Geobacillus kaustophilus ldh* gene, *Thermus thermophilus ldh* gene, *Thermus thermophilus mldh gene,* and *Meiothermus ruber mldh-1 gene and mldh-2 gene.*

The approximately 1.0-kb DNA fragments containing each of *Parageobacillus thermoglucosidasius ldh* gene, *Geobacillus kaustophilus ldh* gene, *Thermus thermophilus ldh* gene, *Thermus thermophilus mldh gene,* and *Meiothermus ruber mldh-1 gene and mldh-2 gene,* that were amplified by the above-described PCR, were cleaved by using restriction enzymes NdeI and EcoRI. The above-mentioned approximately 10.6-kb DNA fragment of cloning vector pCYK21 was also cleaved by using restriction enzymes NdeI and EcoRI. Each of the cleaved 1.0-kb DNA fragments and the 10.6-kb DNA fragment were linked to each other using a T4 DNA Ligase (manufactured by Takara Bio Inc.).

The obtained ligation solutions were used to transform *Hydrogenophilus thermoluteolus* strain TH-1 (NBRC 14978) by electric pulse method, and the obtained transformants were applied onto A-solid medium [(NH₄)₂SO₄ 3.0 g, KH₂PO₄ 1.0 g, K₂HPO₄ 2.0 g, NaCl 0.25 g, FeSO₄▪7H₂O 0.014 g, MgSO₄▪7H₂O 0.5 g, CaCl₂ 0.03 g, MoO₃ 4.0 mg, ZnSO₄▪7H₂O 28 mg, CuSO₄▪5H₂O 2.0 mg, H₃B0₃ 4.0 mg, MnSO₄▪5H₂O 4.0 mg, CoCl₂▪6H₂O 4.0 mg, agar 15g were dissolved in 1 L of distilled water (pH 7.0)] containing kanamycin at 50 µg/ml, and incubated at 50°C for 60 hours in a chamber that was filled with a mixed gas of H₂:O₂:CO₂=7 .5:1:1.5.

Each of the viable strains on the A-solid medium was inoculated using a platinum loop into a test tube containing 5 ml of A-liquid medium [(NH₄)₂SO₄ 3.0 g, KH₂PO₄ 1.0 g, K₂HPO₄ 2.0 g, NaCl 0.25 g, FeSO₄▪7H₂O 0.014 g, MgSO₄▪7H₂O 0.5 g, CaCl₂ 0.03 g, MoO₃ 4.0 mg, ZnSO₄▪7H₂O 28 mg, CuSO₄▪5H₂O 2.0 mg, H₃BO₃ 4.0 mg, MnSO₄▪5H₂O 4.0 mg, CoCl₂▪6H₂O 4.0 mg were dissolved in 1 L of distilled water (pH 7.0)] containing kanamycin at 50 µg/ml. The test tubes were filled with a mixed gas of H₂:O₂:CO₂₌7.5:1:1.5, and subjected to shaking culture at 50°C, and plasmid DNAs were extracted from the culture solution. The plasmids, which comprise *Parageobacillus thermoglucosidasius ldh* gene, *Geobacillus kaustophilus ldh* gene, *Thermus thermophilus ldh* gene, *Thermus thermophilus mldh gene,* and *Meiothermus ruber mldh-1 gene and mldh-2 gene, respectively,* were cleaved using restriction enzymes NdeI and EcoRI, and the inserted fragments were confirmed. As a result, fragments of approximately 1.0-kb in length which were each inserted fragment of *Parageobacillus thermoglucosidasius ldh* gene, *Geobacillus kaustophilus ldh* gene, *Thermus thermophilus ldh* gene, *Thermus thermophilus mldh geneᵣ* and *Meiothermus ruber mldh-1 gene and mldh-2 gene,* in addition to an approximately 10.6-kb DNA fragment of plasmid pCYK21 were observed.

The plasmid containing *Parageobacillus thermoglucosidasius ldh* gene was named as pC-Pth-ldh, the *plasmid containing Geobacillus kaustophilus ldh* gene was named as pC-Gka-ldh, the plasmid containing *Thermus thermophilus ldh* gene was named as pC-Tth-ldh, the plasmid containing *Thermus thermophilus mldh gene* was named as pC-Tth-mldh, the plasmid containing *Meiothermus ruber* mldh-1 *gene* was named as pC-Mru-mldh-1, *and* the plasmid containing *Meiothermus ruber* mldh-2 *gene* was named as pC-Mru-mldh-2.

The plasmids possessed by the recombinant strains of *Hydrogenophilus thermoluteolus* are shown in Table 1.

**[Table 1]**

| Strain | Plasmid | Transgene |
|---|---|---|
| LDH03 | pC-Pth-ldh | ldh (*Parageobacillus thermoglucosidasius*) |
| LDH04 | pC-Gka-ldh | ldh (*Geobacillus kaustophilus*) |
| LDH05 | pC-Tth-ldh | ldh *(Thermus thermophilus)* |
| MLDH01 | pC-Tth-mldh | mldh *(Thermus thermophilus)* |
| MLDH02 | pC-Mru-mldh1 | mldh-1 *(Meiothermus ruber)* |
| MLDH03 | pC-Mru-mldh2 | mldh-2 *(Meiothermus ruber)* |

### (3-2) Confirmation of transgene expression in Hydrogenophilus thermoluteolus strain into which lactic acid producing gene has been introduced

Each lactate dehydrogenase gene or malate/lactate dehydrogenase gene-introduced strain that was obtained as described above, was inoculated using a platinum loop into a test tube containing 5 ml of A-liquid medium containing kanamycin at 50 µg/ml. The test tubes were filled with a mixed gas of H₂:O₂:CO₂=7.5:1:1.5, and subjected to shaking culture at 50°C for 20 hours.

Bacterial cells thus cultured and proliferated were collected by centrifugation (4°C, 15,000 rpm, 1 minute). The bacterial cells were disrupted by sonication, and subsequently centrifuged (4°C, 15,000 rpm, 5 minutes) to obtain a cell disruption supernatant. The cell disruption supernatant was used as a crude enzyme solution to measure lactate dehydrogenase activity by the following method. Crude enzyme solution, 50 mM sodium acetate (pH 5.0), 0.5mM NADH, 0.2 mM fructose 1,6-bisphosphate and 5 mM sodium pyruvate were mixed, reacted at 50°C, and decrease in absorbance at 340nm coming from NADH was traced, and the initial rate of reaction was analyzed. Specific activity was calculated from the initial rate of reaction and protein concentration. The enzyme level for producing 1 µmol of lactic acid per minute was defined as 1 U (Unit).

As a result, lactate dehydrogenase activity of interest was detected in each of strain LDH03 into which *Parageobacillus thermoglucosidasius* ldh gene was introduced, strain LDH04 into which *Geobacillus kaustophilus* ldh gene was introduced, strain LDH05 into which *Thermus thermophilus* ldh gene was introduced, strain MLDH01 into which *Thermus thermophilus* mldh gene was introduced, strain MLDH02 into which *Meiothermus ruber* mldh-1 gene was introduced, and strain MLDH03 into which *Meiothermus ruber* mldh-2 gene was introduced.

**[Table 2]**

| Lactate dehydrogenase activities of *Hydrogenophilus thermoluteolus* strains which are obtained by introducing ldh or mldh gene | | | |
|---|---|---|---|
| Strain | Plasmid | Transgene | Lactate dehydrogenase activity (U/mg-protein) |
| LDH03 | pC-Pth-ldh | ldh (*Parageobacillus thermoglucosidasius*) | 0.55 |
| LDH04 | pC-Gka-ldh | ldh (*Geobacillus kaustophilus*) | 0.14 |
| LDH05 | pC-Tth-ldh | ldh (*Thermus thermophilus*) | 1.21 |
| MLDH01 | pC-Tth-mldh | mldh *(Thermus thermophilus)* | 0.044 |
| MLDH02 | pC-Mru-mldh1 | mldh-1 *(Meiothermus ruber)* | 0.24 |
| MLDH03 | pC-Mru-mldh2 | mldh-2 *(Meiothermus ruber)* | 0.021 |
| pCYK21 /TH-1 | pCYK21 | None | ND(Undetectable) |

### (3-3) Production of lactic acid

*Hydrogenophilus thermoluteolus* strain into which lactate dehydrogenase gene was introduced, was inoculated using a platinum loop into A-liquid medium containing kanamycin at 50 µg/ml, and subjected to shaking culture at 50°C for 30 hours while supplying a mixed gas of H₂:O₂:CO₂₌7.5:1:1.5 during incubation.

Following incubation, a culture supernatant was obtained by centrifugation (4°C, 15,000 rpm, 1 minute), and lactic acid in the culture supernatant was quantified. As a result, lactic acid was produced in the culture supernatant, as shown in Table 3.

**[Table 3]**

| Strain | Plasmid | Transgene | Lactic acid concentration in culture supernatant (mM) |
|---|---|---|---|
| LDH03 | pC-Pth-ldh | ldh (*Parageobacillus thermoglucosidasius*) | 1.2 |
| LDH04 | pC-Gka-ldh | ldh (*Geobacillus kaustophilus*) | 0.7 |
| LDH05 | pC-Tth-ldh | ldh *(Thermus thermophilus)* | 1.8 |
| MLDH01 | pC-Tth-mldh | mldh (*Thermus thermophilus*) | 0.6 |
| MLDH02 | pC-Mru-mldh1 | mldh-1 *(Meiothermus ruber)* | 1.5 |
| MLDH03 | pC-Mru-mldh2 | mldh-2 *(Meiothermus ruber)* | 0.4 |
| pCYK21/TH-1 | pCYK21 | None | 0.2 |

### (4) Deposited strains

*Hydrogenophilus thermoluteolus* LDH05 strain and *Hydrogenophilus thermoluteolus* MLDH02 strain were deposited to NITE Patent Microorganisms Depositary, National Institute of Technology and Evaluation (2-5-8 Kazusakamatari, Kisarazu-shi, Chiba, Japan (postal code 292-0818)). For *Hydrogenophilus thermoluteolus* LDH05 strain, the accession number is BP-02822 and the date of acceptance is November 14, 2018. For *Hydrogenophilus thermoluteolus* MLDH02 strain, the accession number is BP-02828 and the date of acceptance is November 21, 2018. Accordingly, these strains are available to the public.

Furthermore, all strains (including ATCC strains and NBRC strains) that are described in the present specification are internationally deposited under the Budapest Treaty, or are possessed by organizations that furnish the strains without any terms or conditions, or are marketed, and therefore, these strains are all available to the general public.

### Industrial Applicability

The transformant of the present invention effectively produces lactic acid using carbon dioxide as a sole carbon source, and therefore, it is able to efficiently produce biodegradable plastics, while solving global warming caused by increased emissions of carbon dioxide.

## Claims

1. A transformant obtained by introducing (a) a lactate dehydrogenase gene and/or (b) a malate/lactate dehydrogenase gene into a *Hydrogenophilus* bacterium.

2. The transformant according to claim 1, wherein (a) the lactate dehydrogenase gene is the following DNA (a1), (a2), (a3), (a4), (a5) or (a6):
(a1) DNA which consists of a base sequence of SEQ ID NO: 1, 2 or 3;
(a2) DNA which consists of a base sequence having 90% or more identity to SEQ ID NO: 1, 2 or 3, the DNA encoding a polypeptide having lactate dehydrogenase activity;
(a3) DNA which hybridizes with a DNA consisting of a base sequence complementary to SEQ ID NO: 1, 2 or 3 under stringent conditions, and which encodes a polypeptide having lactate dehydrogenase activity;
(a4) DNA which encodes a polypeptide consisting of an amino acid sequence of SEQ ID NO: 4, 5 or 6;
(a5) DNA which encodes a polypeptide consisting of an amino acid sequence having 90% or more identity to SEQ ID NO: 4, 5 or 6, the polypeptide having lactate dehydrogenase activity;
(a6) DNA which encodes a polypeptide consisting of an amino acid sequence having a deletion, substitution, or addition of one or a plurality of amino acids in an amino acid sequence of SEQ ID NO: 4, 5 or 6, the polypeptide having lactate dehydrogenase activity.

3. The transformant according to claim 1 or 2, wherein (b) the malate/lactate dehydrogenase gene is the following DNA (b1), (b2), (b3), (b4), (b5) or (b6):
(b1) DNA which consists of a base sequence of SEQ ID NO: 7, 8 or 9;
(b2) DNA which consists of a base sequence having 90% or more identity to SEQ ID NO: 7, 8 or 9, the DNA encoding a polypeptide having lactate dehydrogenase activity;
(b3) DNA which hybridizes with a DNA consisting of a base sequence complementary to SEQ ID NO: 7, 8 or 9 under stringent conditions, and which encodes a polypeptide having lactate dehydrogenase activity;
(b4) DNA which encodes a polypeptide consisting of an amino acid sequence of SEQ ID NO: 10, 11 or 12;
(b5) DNA which encodes a polypeptide consisting of an amino acid sequence having 90% or more identity to SEQ ID NO: 10, 11 or 12, the polypeptide having lactate dehydrogenase activity;
(b6) DNA which encodes a polypeptide consisting of an amino acid sequence having a deletion, substitution, or addition of one or a plurality of amino acids in the amino acid sequence of SEQ ID NO: 10, 11 or 12, the polypeptide having lactate dehydrogenase activity.

4. The transformant according to any one of claims 1-3, wherein the *Hydrogenophilus* bacterium is *Hydrogenophilus thermoluteolus.*

5. A method for producing lactic acid comprising a step of culturing the transformant according to any one of claims 1-4 through use carbon dioxide as a substantially sole carbon source.
